# FASCICULE DE BREVET EUROPEEN

(11) **EP 0 593 313 B1**
(45) Date de publication et mention de la délivrance du brevet: **15.01.1997**
(21) Numéro de dépôt: 93400881.4
(22) Date de dépôt: 05.04.1993
(51) Int. Cl.: C07C 319/28, C07C 323/52

(54) **Procédé de désodorisation d'un mercaptoacide par extraction des composés malodorants et produit désodorisé ainsi obtenu**
Verfahren zur Deodorisierung von einer Mercaptosäure durch Extraktion von übelriechenden Verbindungen sowie damit erhältliches, deodoriertes Produkt
Method of deodorising a mercapto acid by extraction of malodorous compounds and deodorised product thus obtained

(30) Priorité: 15.10.1992 FR 9212345
(43) Date de publication de la demande: 20.04.1994
(73) Titulaire: L'OREAL, 75008 Paris (FR)
(72) Inventeur: Dedieu, Michel, F-78600 Maisons Laffite (FR); Burgaud, Hervé, F-77230 Dammartin en Goele (FR); Lapoirie, Eric, F-93250 Villemomble (FR); Gurfein, Véronique, F-92350 Le Plessis Robinson (FR); Malle, Gérard, F-77580 Villiers s/Morin (FR)
(74) Mandataire: Peuscet, Jacques

(56) Documents cités:
- PATENT ABSTRACTS OF JAPAN vol. 8, no. 114 (C-225)26 Mai 1984 & JP-A-57 027 866 (RAION) 14 Février 1984

## Description

La présente invention concerne un procédé de désodorisation d'un mercaptoacide par extraction des composés malodorants et le produit désodorisé ainsi obtenu.

Les mercaptoacides sont des composés bien connus, qui ont une fonction thiol (-SH) et une fonction acide (- COOH). Ils ont de nombreuses applications. L'acide thioglycolique (ou mercaptoacétique), par exemple, peut être utilisé, sous sa forme acide, comme intermédiaire dans la synthèse de nombreux pesticides et produits pharmaceutiques, ou, sous sa forme acide ou salifiée (en particulier, sel d'ammonium, d'amine, de sodium, de potassium ou de calcium), pour le décapage de surfaces métalliques, le traitement de minerais sulfurés, et le traitement des cuirs et peaux ; dans l'industrie cosmétique, il constitue également l'agent réducteur le plus utilisé pour la déformation permanente des cheveux (frisage ou défrisage) et la matière active principale des laits et crèmes dépilatoires. De même, l'acide thiolactique (acide 2-mercaptopropionique) est utilisé comme réducteur pour la déformation permanente des cheveux ou comme constituant des laits et crèmes dépilatoires.

Les mercaptoacides purs ont une légère odeur piquante qui n'est pas vraiment déplaisante. Mais ils contiennent toujours des composés sulfurés, tels que l'hydrogène sulfuré et les mercaptans de faible poids moléculaire, en particulier le méthanethiol ou l'éthanethiol, qui ont une odeur nauséabonde particulièrement désagréable. Il suffit de très faibles quantités de ces composés sulfurés, pour que l'on perçoive leur présence à l'odorat, le nez étant dans ce cas le meilleur instrument de détection.

La présence de ces composés malodorants est liée à divers processus de décomposition des mercaptoacides, processus qui sont encore très mal connus mais sont, sans doute, dus à la fois à des mécanismes ioniques et radicalaires pouvant se produire à l'abri de l'air. Cette décomposition et la formation de composés malodorants, qui en résulte, peuvent, d'ailleurs, être suivies au cours du temps par différentes techniques analytiques, en particulier par la méthode dite du "head space" en chromatographie gazeuse.

Dans les différentes applications, et plus particulièrement dans leurs applications cosmétiques, l'odeur dégagée par les mercaptoacides constitue une réelle gêne pour les utilisateurs. On a donc essayé de masquer l'odeur des mercaptoacides par des parfums, mais, en général, cette odeur est trop puissante pour pouvoir être masquée de façon satisfaisante. On a également proposé, dans la demande de brevet japonais n° 82-136 280 publiée sous le n° 84/027 866, de désodoriser l'acide thioglycolique pur ou en mélange avec de l'eau, par extraction par un hydrocarbure non aromatique en C₄-C₈. Mais on a constaté que, si ce procédé d'extraction permet d'extraire les composés malodorants et d'obtenir un acide désodorisé, l'effet de désodorisation obtenu n'est pas durable dans le temps car les composés malodorants se reforment rapidement et annulent le bénéfice obtenu par le traitement ; dans certains cas, l'odeur revient même à un niveau supérieur au niveau initial.

L'invention concerne un procédé d'extraction des composés malodorants des mercaptoacides, qui permet d'obtenir un mercaptoacide désodorisé, l'effet de désodorisation étant durable.

La présente invention a donc pour objet un procédé de désodorisation d'un mercaptoacide par extraction de la majeure partie des produits de décomposition malodorants contenus dans le mercaptoacide soumis au traitement, caractérisé par le fait que le mercaptoacide traité a pour formule :

HS - A - COOH (I)

formule dans laquelle A représente :
- le radical divalent

   -(CH₂)ₙ-

   où n est un nombre entier compris entre 1 et 4,
- le radical divalent 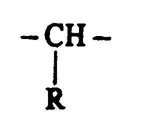 où R représente un radical alkyle, linéaire ou ramifié, en C₁ - C₃, ou
- le radical divalent 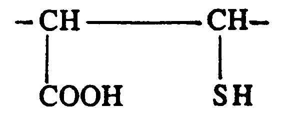 et que l'on effectue l'extraction à l'aide de dioxyde de carbone à l'état gazeux, liquide ou solide.

Le mercaptoacide traité est, de préférence, l'acide thioglycolique ou l'acide thiolactique ; on peut cependant citer également l'acide 4-mercapto butyrique, l'acide 3-mercapto propionique et l'acide dimercaptosuccinique.

Selon ce procédé d'extraction, le gaz carbonique se charge en composés malodorants et on obtient, comme résidu liquide d'extraction, le mercaptoacide désodorisé. On a constaté que le mercaptoacide obtenu était entièrement désodorisé : en effet, il ne présente plus que son odeur légèrement piquante propre ; de plus, un dosage du sulfure d'hydrogène et de méthanethiol par chromatographie gazeuse dite "head space" montre que ces composés ont été totalement éliminés. Par ailleurs, on a constaté, de façon tout à fait inattendue, qu'après deux mois de conservation du mercaptoacide désodorisé à température ambiante, à l'abri de l'air, l'odorat humain ne permet pas de déceler la présence de composés malodorants. Par conséquent, ceux-ci ne se sont pas reformés ou ne se sont reformés que dans des proportions très faibles. Il faut noter que le mercaptoacide désodorisé n'est pas nécessairement pur : il pourrait contenir des impuretés non odorantes.

L'extraction est effectuée par contact du mercaptoacide, éventuellement en solution, avec le dioxyde de carbone gazeux, liquide ou solide. Le dioxyde de carbone solide est, en particulier, sous forme de "CARBOGLACE". Il faut noter qu'une partie de ce dioxyde de carbone peut, éventuellement, se trouver, à un moment donné, à l'état supercritique au cours du procédé d'extraction.

Les températures et les pressions d'extraction utilisées sont choisies de façon à maintenir tout ou partie de l'anhydride carbonique en dehors du domaine supercritique borné par le point critique de température qui est de 31° C et le point critique de pression qui est de 73,8 x 10⁵ pascals

Les mercaptoacides étant, en général, des produits liquides à température ambiante, on peut effectuer l'extraction par contact direct de l'acide avec CO₂. Cependant, on peut utiliser une solution de mercaptoacide dans un solvant, en particulier un solvant polaire.

Le solvant polaire est, de préférence, choisi dans le groupe formé par l'eau, les monoalcools linéaires ou ramifiés en C₁ - C₅, les diols en C₂ - C₆, les polyols en C₃ - C₆ et leurs mélanges. L'eau est le solvant préféré car, dans la plupart des applications, son élimination ultérieure n'est pas nécessaire.

Le monoalcool peut être choisi dans le groupe formé par le méthanol, l'éthanol, le 1-propanol, le 2-propanol, le 1-butanol, le 2-butanol, le 2-méthyl propanol ou le 1-pentanol.

Le diol est avantageusement choisi dans le groupe formé par l'éthylèneglycol, le 1,2-propane diol, le 1,3-propanediol, le 1,3-butanediol, le 2,3-butanediol et le 1,6-hexanediol.

Le polyol peut avantageusement être le glycérol.

Dans le cas où le mercaptoacide est sous forme de solution, le solvant peut être utilisé en quantités comprises entre 1 et 99 % en poids par rapport au mercaptoacide. On utilise, de préférence, des solutions aqueuses, alcooliques ou hydroalcooliques ayant une concentration en mercaptoacide comprise entre 0,1 mole/litre et 14 moles/litre et, de préférence, entre 1 mole/litre et 10 moles/litre.

L'extraction peut être effectuée en présence d'un composé gazeux inerte tel que l'hélium, l'azote ou l'argon. On améliore ainsi le rendement d'extraction par rapport à la quantité de CO₂ mise en oeuvre.

Lorsque le dioxyde de carbone est sous forme de gaz ou de liquide, l'extraction peut être effectuée aussi bien en continu qu'en semi-continu et en discontinu. Lorsque le dioxyde de carbone est sous forme solide on opère, de préférence, en discontinu.

Lorsqu'on opère en discontinu, on introduit, dans un réacteur, le mercaptoacide, éventuellement un solvant, le dioxyde de carbone sous forme gazeuse, liquide ou solide, et, éventuellement, un gaz inerte ; on fait en sorte que les conditions de pression et de température désirées pour l'extraction soient obtenues. On laisse en contact l'ensemble pendant 5 à 30 minutes puis on soutire le dioxyde de carbone. On effectue éventuellement deux ou plusieurs cycles d'extraction en réintroduisant du dioxyde de carbone, en rétablissant les conditions de température et pression désirées pour l'extraction, en laissant en contact l'ensemble et en évacuant le CO₂. A la fin du dernier cycle, on recueille le mercaptoacide désodorisé ou une solution de mercaptoacide désodorisé.

Lorsqu'on opère en semi-continu, on introduit dans un réacteur le mercaptoacide et, éventuellement, le solvant, on remplit le réacteur de dioxyde de carbone et on fait en sorte que l'on obtienne les conditions de pression et température désirées pour l'extraction. On assure ensuite un débit de lavage par le dioxyde de carbone gazeux ou liquide pendant une durée déterminée, débit qui peut être constant ou variable, ce débit étant fonction de l'installation. Ce débit est, généralement, compris entre 0,5 et 3 kg/h. Il est souvent préférable de rapporter ce débit à la quantité de produit traité : il est généralement compris entre 1 et 30 kg/h par kg de mélange soumis à l'extraction. Pour finir on soutire le dioxyde de carbone et on recueille le mercaptoacide désodorisé ou une solution de mercaptoacide désodorisé.

Lorsqu'on opère en continu, on réalise l'extraction du mercaptoacide par le dioxyde de carbone sous forme liquide ou gazeuse à courants parallèles ou à contre-courant sur une colonne de séparation. On assure à la fois un débit continu du mélange à extraire et du dioxyde de carbone. Le rapport des deux débits est fixé de façon à obtenir le rendement d'extraction le meilleur possible.

Après séparation du dioxyde de carbone, le produit désodorisé recueilli est constitué par le mercaptoacide et, éventuellement, la majeure partie du solvant ; il contient probablement de faibles quantités de CO₂ résiduel. Il faut noter qu'il peut contenir, sans inconvénients, des impuretés non odorantes.

Le produit désodorisé obtenu est, de préférence, soigneusement conditionné à l'abri de l'oxygène de l'air dans un conteneur connu pour sa compatibilité chimique avec le mercaptoacide sans éliminer le dioxyde de carbone résiduel. Dans ces conditions, il peut être conservé pendant des mois sans que l'odeur désagréable due à l'hydrogène sulfuré ou aux thiols de décomposition ne réapparaisse de façon décelable à l'odorat.

Le solvant, en particulier le solvant alcoolique, peut éventuellement être éliminé, au moins partiellement, du produit désodorisé, par tout procédé connu, avant stockage.

Quel que soit le procédé d'extraction utilisé, le dioxyde de carbone peut être recyclé après avoir été débarrassé des composés maladorants extraits selon des méthodes connues telles que le passage sur charbon actif.

Les mercaptoacides de formule (I) ou solutions de mercaptoacide de formule (I) désodorisés obtenus peuvent être utilisés dans toutes les applications industrielles connues pour ces produits. Le mercaptoacide est généralement associé avec d'autres ingrédients dans une composition dont la formulation varie selon l'application. L'efficacité de la désodorisation permet de les utiliser, sans donner d'odeur désagréable, dans toute composition qui ne doit pas avoir d'odeur désagréable et dont les autres ingrédients ne possèdent pas d'odeur désagréable. On peut mentionner, à titre d'exemple, la possibilité d'association avec des thiols tels que la cystéine ou la cystéamine qui ne présentent pas d'odeur désagréable.

Lorsque la composition utilisée contient des ingrédients susceptibles de faire perdre le bénéfice de la désodorisation en provoquant la formation de composés malodorants, il peut être avantageux de conditionner le mercaptoacide ou la solution de mercaptoacide obtenu, séparément et de préparer la quantité de composition nécessaire à l'application, au dernier moment.

Les exemples donnés ci-après, à titre illustratif et non limitatif, permettront de mieux comprendre l'invention.

Les exemples 1 à 3 sont des exemples de désodorisation.

### EXEMPLE 1

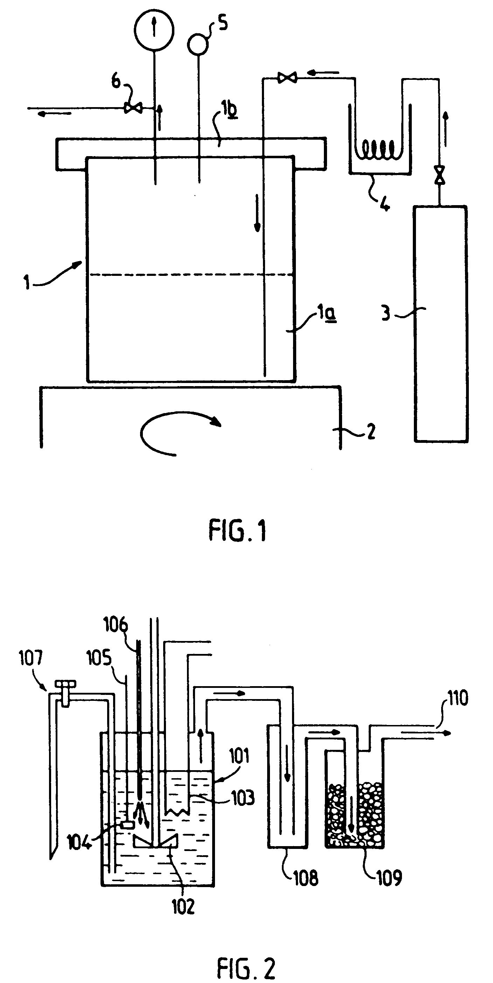

Dans cet exemple, l'extraction a été effectuée dans une installation d'extraction en discontinu schématisée sur la figure 1 annexée.

L'installation comporte un réacteur 1 ayant une capacité de 100 ml constitué d'une enveloppe d'acier 1a résistant à la pression et d'un couvercle 1b. Le réacteur 1 est muni d'un système d'agitation et de chauffage schématisé en 2. Le dioxyde de carbone est stocké sous forme liquide sous pression dans un réservoir 3 qui est relié au réacteur par l'intermédiaire d'un condenseur 4 qui permet de liquéfier le dioxyde de carbone. Le réacteur 1 est muni d'une soupape de sécurité 5 ; il est relié par l'intermédiaire d'une vanne 6 à un restricteur (non représenté sur la figure) constitué par un tube de silice de 30 cm de long et de 50 microns de diamètre interne qui assure la décompression lente du réacteur.

On introduit dans le réacteur 1, 50 ml d'une solution aqueuse d'acide thioglycolique à 92 g/l (1M). Le réacteur est alors rempli de dioxyde de carbone liquide provenant du réservoir 3 à la température de 10° C et sous une pression de 45 x 10⁵ pascals. On isole le réacteur et on met l'agitation en marche. On laisse la température s'élever jusqu'à la température ambiante (23° C). La pression atteint alors 60 x 10⁵ pascals. On maintient le contact sous agitation pendant 20 minutes. Le dioxyde de carbone est ensuite éliminé par décompression lente à travers le restricteur. Lorsque la pression dans le réacteur atteint 50 x 10⁵ pascals, on ramène la pression à 60 x 10⁵ pascals et la température à 23° C, et on maintient à nouveau l'ensemble sous agitation pendant 20 minutes. On effectue ensuite une décompression complète du réacteur et on recueille une solution aqueuse d'acide thioglycolique désodorisé. On ne décèle aucune odeur et une analyse par chromatographie gazeuse montre qu'il n'y a ni hydrogène sulfuré, ni méthanethiol dans l'acide.

On constate qu'après 1 mois de stockage à l'abri de l'air, l'acide thioglycolique n'a pas repris d'odeur décelable à l'odorat.

### EXEMPLE 2

On introduit, dans un réacteur de 100 ml muni d'un agitateur, 50 ml d'une solution d'acide thioglycolique à 184 g/l (2M) et on chauffe le mélange à 50° C. On introduit ensuite progressivement, sous agitation énergique, 100 g de "CARBOGLACE" de façon que la température initiale ne varie pas. Lorsque la totalité de la "CARBOGLACE" est introduite, on laisse le mélange refroidir à température ordinaire, sous couverture de CO₂.

L'acide thioglycolique obtenu ne possède plus l'odeur désagréable qu'il présentait avant traitement.

### EXEMPLE 3

Dans cet exemple, l'extraction est effectuée dans l'installation illustrée sur la figure 2.

Cette installation comprend un réacteur 101 de 0,5 litre équipé d'un agitateur 102, magnétique ou mécanique, et d'un chauffage par thermoplongeur 103. Un thermocouple 104, relié à un dispositif de mesure de la température 105, permet une régulation de la température. Un conduit 106 permet d'introduire de l'anhydride carbonique CO₂ provenant d'une source non représentée. Sur la partie haute du réacteur 101 est disposé un conduit de soutirage du gaz contenu dans le réacteur qui permet le passage du gaz dans un piège statique 108. Le piège 108 est lui-même relié en partie haute à un piège à charbon actif 109 pour capter les composés malodorants. Les effluents gazeux sont soutirés en partie haute du piège 109 par un conduit 110. Le liquide contenu dans le réacteur 101 est soutiré du réacteur 101 par un tube 107 qui plonge dans ledit réacteur et qui est muni d'un robinet.

On introduit, dans le réacteur 101, 250 ml d'une solution aqueuse d'acide thioglycolique à 368 g/l (4M) et, après mise en route de l'agitation, on porte la température à 40°C. On injecte ensuite l'anhydride carbonique dans la solution sous une pression de 250 x 10⁵ pascals et avec un débit de 3 ml/minute. On soutire en 110 l'effluent gazeux ne contenant pratiquement plus de composés malodorants. Au bout de 3 heures, on refroidit le réacteur à 20°C et on soutire la solution aqueuse d'acide désodorisé par le conduit 107.

L'acide thioglycolique obtenu ne présente plus l'odeur désagréable qu'il présentait avant traitement.

Les exemples 4 à 11 sont des exemples d'utilisation d'une solution aqueuse d'un mercaptoacide de formule I désodorisé.

### EXEMPLE 4

On prépare :

### A - Une composition réductrice :

Cette préparation se fait à partir de deux solutions stockées dans des flacons différents.

### Solution (a) :

| | |
|---|---|
| - Solution aqueuse d'acide thioglycolique désodorisé à 36 % de matière active | 25 g |

### Solution (b) :

| | |
|---|---|
| - Oxyde de laurylamine commercialisé sous la dénomination "AROMOX DMMCD/W" par la société "AKZO" | 2 g |
| - Acide éthylènediamine tétraacétique | 0,15 g |
| - Monoéthanolamine qsp | pH = 9,0 |
| - Eau déminéralisée qsp | 75 g |

Après mélange de la solution (a) avec la solution (b), on obtient une composition réductrice ayant un pH de 9,0.

On applique cette composition réductrice sur des cheveux mouillés, préalablement enroulés sur des bigoudis, puis on recouvre les cheveux d'un bonnet en matière plastique et on laisse agir pendant 15 minutes à température ambiante. On rince ensuite abondamment les cheveux à l'eau et on applique alors la composition oxydante définie en B.

### B - Composition oxydante ayant la formulation suivante :

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

On laisse agir la composition oxydante pendant 5 minutes puis on déroule les cheveux et laisse encore agir pendant 3 minutes. On rince enfin abondamment à l'eau.

Après séchage sous casque, on constate que les cheveux présentent de belles boucles avec un bon degré de frisure.

On a constaté, à l'odorat, qu'au cours de l'opération, il ne se développe pas d'odeur nauséabonde.

### EXEMPLE 5

En utilisant le même mode opératoire que celui décrit dans l'exemple 4, on a procédé à une déformation permanente des cheveux à l'aide des compositions réductrice et oxydante suivantes :

### A - Composition réductrice

### - Solution (a)

| | |
|---|---|
| - Solution aqueuse d'acide thioglycolique désodorisé à 18 % de matière active | 50 g |

### Solution (b) :

| | |
|---|---|
| - Oxyde de laurylamine commercialisé sous la dénomination "AROMOX DMMCD/W" par la société "AKZO" | 0,9 g |
| - Sel pentasodique de l'acide diéthylène triamine pentaacétique | 0,15 g |
| - Parfum qs | |
| - Ammoniaque (20 % dans l'eau) qsp | pH = 7,8 |
| - Eau déminéralisée qsp | 50 g |

On obtient par mélange une composition ayant un pH de 7,8.

### B - Composition oxydante

On utilise la même composition oxydante que celle décrite dans l'exemple 4.

On a constaté, à l'odorat, qu'au cours de l'opération, il ne se développe pas d'odeur nauséabonde.

### EXEMPLE 6

En utilisant le même mode opératoire que celui décrit dans l'exemple 4, on a procédé à une déformation permanente des cheveux, à l'aide des compositions réductrice et oxydante suivantes :

### A - Composition réductrice

### - Solution (a)

| | |
|---|---|
| - Solution aqueuse d'acide thiolactique désodorisé à 22 % de matière active | 50 g |

### Solution (b) :

| | |
|---|---|
| - Oxyde de laurylamine commercialisé sous la dénomination "AROMOX DMMCD/W" par la société "AKZO" | 0,9 g |
| - Parfum qs | |
| - Ammoniaque (20 % dans l'eau) qsp | pH = 8,2 |
| - Eau déminéralisée qsp | 50 g |

On obtient par mélange une composition ayant un pH de 8,2.

### B - Composition oxydante

On utilise la même composition oxydante que celle décrite à l'exemple 4.

On a constaté, à l'odorat, qu'au cours de l'opération, il ne se développe pas d'odeur nauséabonde.

### EXEMPLE 7

En utilisant le même mode opératoire que celui décrit dans l'exemple 4, on a procédé à une déformation permanente des cheveux, à l'aide des compositions réductrice et oxydante suivantes :

### A - Composition réductrice

### - Solution (a)

| | |
|---|---|
| - Solution aqueuse d'acide thioglycolique désodorisé à 18 % de matière active | 50 g |

### Solution (b) :

| | |
|---|---|
| - Cystéine | 4,5 g |
| - Ester stéarique polyoxyéthyléné avec 8 moles d'oxyde d'éthylène commercialisé sous la dénomination "MYRJ 45" par la société "ICI" | 0,85 g |
| - Conservateur | 0,35 g |
| - Parfum qs | |
| - Ammoniaque qsp | pH = 8,8 |
| - Eau déminéralisée qsp | 50 g |

On obtient par mélange une composition ayant un pH de 8,8.

### B - Composition oxydante

| | |
|---|---|
| - Eau oxygénée (20 volumes) | 40 g |
| - Acide citrique qsp | pH = 3,2 |
| - Eau déminéralisée qsp | 100 g |

On a constaté, à l'odorat, qu'au cours de l'opération, il ne se développe pas d'odeur nauséabonde.

### EXEMPLE 8

En utilisant le même mode opératoire que celui décrit dans l'exemple 4, on a procédé à une déformation permanente des cheveux, à l'aide des compositions réductrice et oxydante suivantes :

### A - Composition réductrice

### - Solution (a)

| | |
|---|---|
| - Solution aqueuse d'acide thioglycolique désodorisé à 18 % de matière active | 40 g |

### Solution (b) :

| | |
|---|---|
| - Chlorhydrate de cystéamine | 6,0 g |
| - Oxyde de laurylamine commercialisé sous la dénomination "AROMOX DMMCD/W" par la société "AKZO" | 2 g |
| - Conservateur | 0,15 g |
| - Parfum qs | |
| - Monoéthanolamine qsp | pH = 7,8 |
| - Eau déminéralisée qsp | 60 g |

On obtient par mélange une composition ayant un pH de 7,8.

### B - Composition oxydante

| | |
|---|---|
| - Bromate de sodium | 8 g |
| - Triéthanolamine qsp | pH = 8 |
| - Phosphate monosodique hydraté (12H₂0) | 0,3 g |
| - Phosphate trisodique hydraté | 0,5 g |
| - Cocoamidopropylbétaïne commercialisé sous la dénomination "TEGOBETAINE HS" par la société "GOLDSCHMIDT" | 1 g |
| - Parfum qs | |
| - Eau déminéralisée qsp | 100 g |

On a constaté, à l'odorat, qu'au cours de l'opération, il ne se développe pas d'odeur nauséabonde.

### EXEMPLE 9

On a utilisé :

### A - Une composition réductrice

stockée dans un seul flacon et ayant la composition suivante :

| | |
|---|---|
| - Solution aqueuse d'acide thioglycolique désodorisé à 36 % de matière active | 25 g |
| - Oxyde de laurylamine commercialisé sous la dénomination "AROMOX DMMCD/W" par la société "AKZO" | 2 g |
| - Acide éthylènediamine tétraacétique | 0,15 g |
| - Monoéthanolamine qsp | pH = 9 |
| - Eau déminéralisée qsp | 100 g |

On applique cette composition réductrice sur des cheveux mouillés, préalablement enroulés sur des bigoudis, puis on recouvre les cheveux d'un bonnet en matière plastique et on laisse ensuite agir pendant 15 minutes à température ambiante. On rince alors abondamment les cheveux à l'eau et on applique la composition oxydante suivante :

### B - Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

On a constaté, à l'odorat, qu'au cours de l'opération, il ne se développe pas d'odeur nauséabonde.

### EXEMPLE 10

On prépare :

### A - Une composition réductrice de défrisage à partir de deux solutions conservées dans des flacons différents :

### - Solution (a)

| | |
|---|---|
| - Solution aqueuse d'acide thioglycolique désodorisé à 18 % de matière active | 50 g |

### Solution (b) :

| | |
|---|---|
| - Acide polyacrylique commercialisé sous la dénomination "CARBOPOL 934" par la société "GOODRICH" | 8 g |
| - Ammoniaque (20 % dans l'eau) qsp | pH = 8,2 |
| - Parfum qs | |
| - Eau déminéralisée qsp | 50 g |

Après mélange de la solution (a) avec la solution (b), on obtient une composition réductrice de défrisage ayant un pH de 8,2.

On applique cette composition sur des cheveux mouillés, initialement bouclés, et on lisse les cheveux, de façon à les rendre raides, à l'aide d'un peigne. Après un temps de pose de 10 minutes, on rince abondamment les cheveux à l'eau et on applique la composition oxydante suivante :

### B - Composition oxydante

| | |
|---|---|
| - Eau oxygénée | 2 g |
| - Stannate de sodium | 0,015 g |
| - Lauryl sulfate d'ammonium | 1,4 g |
| - Hydrolysat de protéines | 0,6 g |
| - Acide citrique | 0,5 g |
| - Parfum qs | |
| - Eau déminéralisée qsp | 100 g |

On laisse agir la composition oxydante pendant 5 minutes puis on rince abondamment à l'eau.

On a constaté, à l'odorat, qu'au cours de l'opération, il ne se développe pas d'odeur nauséabonde.

### EXEMPLE 11

On prépare une **composition dépilatoire** à partir de deux solutions conservées dans des flacons différents :

### - Solution (a)

| | |
|---|---|
| - Solution aqueuse d'acide thioglycolique désodorisé à 18 % de matière active | 20 g |

### Solution (b) :

| | |
|---|---|
| - Urée | 5 g |
| - Carbonate de calcium | 1 g |
| - Alcool cétylstéarylique et cétylstéarylique oxyéthyléné (mélange 80/20) | 10 g |
| - Parfum qs | |
| - Eau déminéralisée qsp | 80 g |

On mélange la solution (a) avec la solution (b), et on ajuste le pH à 11,2 par ajout d'hydroxyde de calcium.

On applique cette composition sur la peau et on laisse agir pendant 10 minutes puis on rince abondamment à l'eau.

On a constaté, à l'odorat, qu'au cours de l'opération, il ne se développe pas d'odeur nauséabonde.

## Revendications

1. Procédé de désodorisation d'un mercaptoacide par extraction de la majeure partie des produits de décomposition malodorants contenus dans le mercaptoacide soumis au traitement, caractérisé par le fait que le mercaptoacide traité a pour formule :
HS - A - COOH (I)
formule dans laquelle A représente :
- le radical divalent
-(CH₂)ₙ-
où n est un nombre entier compris entre 1 et 4,
- le radical divalent 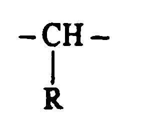 où R représente un radical alkyle linéaire ou ramifié en C₁ - C₃, ou
- le radical divalent 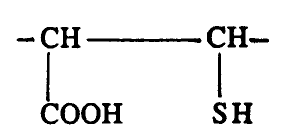 et que l'on effectue l'extraction à l'aide de dioxyde de carbone à l'état gazeux, liquide ou solide.

2. Procédé selon la revendication 1, caractérisé par le fait que le mercaptoacide traité est l'acide thioglycolique ou l'acide thiolactique.

3. Procédé selon l'une des revendications 1 ou 2, caractérisé par le fait que l'extraction est effectuée à une température et une pression hors du domaine supercritique.

4. Procédé selon l'une des revendications 1 à 3, caractérisé par le fait qu'on effectue l'extraction par contact direct de l'acide avec le dioxyde de carbone.

5. Procédé selon l'une des revendications 1 à 4, caractérisé par le fait qu'on effectue l'extraction sur une solution de mercaptoacide dans un solvant polaire.

6. Procédé selon la revendication 5, caractérisé par le fait que le solvant polaire est choisi dans le groupe forme par l'eau, les monoalcools linéaires ou ramifiés en C₁-C₅, les diols en C₂-C₆, les polyols en C₃-C₆.

7. Procédé selon l'une des revendications 5 ou 6, caractérisé par le fait que le solvant est utilisé en une quantité comprise entre 1 et 99 % en poids par rapport au mercaptoacide.

8. Procédé selon l'une des revendications 5 à 7, caractérisé par le fait que la solution de mercaptoacide a une concentration en mercaptoacide comprise entre 0,1 mole/litre et 14 moles/litre.

9. Procédé selon l'une des revendications 1 à 8, caractérisé par le fait que l'extraction est effectuée en présence d'un composé gazeux inerte.

10. Procédé selon l'une des revendications 1 à 9, caractérisé par le fait qu'on introduit, dans un réacteur, un mercaptoacide, éventuellement un solvant, le dioxyde de carbone sous forme gazeuse, liquide ou solide, et, éventuellement, un gaz inerte, on fait en sorte d'obtenir les conditions de pression et de température désirées pour l'extraction, on laisse en contact l'ensemble pendant 5 et 30 minutes, on soutire le dioxyde de carbone, on effectue éventuellement plusieurs cycles d'extraction en réintroduisant du dioxyde de carbone, en rétablissant les conditions de température et pression désirées pour l'extraction en laissant en contact l'ensemble et en évacuant le CO₂, et on recueille le mercaptoacide désodorisé ou une solution de mercaptoacide désodorisé.

11. Procédé selon l'une des revendications 1 à 9, caractérisé par le fait qu'on introduit dans un réacteur le mercaptoacide et éventuellement un solvant, on remplit le réacteur de dioxyde de carbone et on fait en sorte d'obtenir les conditions de pression et de température désirées pour l'extraction, on assure un débit de lavage par le dioxyde de carbone gazeux ou liquide pendant une durée déterminée, on évacue le CO₂ et on recueille le mercaptoacide désodorisé ou une solution de mercaptoacide désodorisé.

12. Procédé selon la revendication 11, caractérisé par le fait que le débit de dioxyde de carbone est compris entre 0,5 et 3 kg/h.

13. Procédé selon la revendication 12, caractérisé par le fait que le débit de dioxyde de carbone est compris entre 1 et 30 kg/h par kg de mélange soumis à l'extraction.

14. Procédé selon l'une des revendications 1 à 9, caractérisé par le fait qu'on effectue l'extraction en continu par le dioxyde de carbone sous forme liquide ou gazeuse sur une colonne de séparation.

15. Procédé selon l'une des revendications 10 à 14, caractérisé par le fait que le dioxyde de carbone est recyclé après avoir été débarrassé des composés malodorants.

## Claims

1. Process for deodorising a mercapto acid by extraction of the greater part of the malodorous decomposition products contained in the mercapto acid subjected to the treatment, characterised in that the mercapto acid treated is of the formula:
HS - A - COOH (I)
in which formula A represents
- the divalent radical
-(CH₂)ₙ-
where n is an integer between 1 and 4,
- the divalent radical 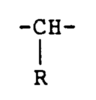 where R represents a linear or branched C₁-C₃ alkyl radical, or
- the divalent radical 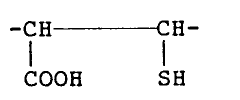 and in that the extraction is performed using carbon dioxide in the gaseous, liquid or solid state.

2. Process according to Claim 1, characterised in that the mercapto acid treated is thioglycolic acid or thiolactic acid.

3. Process according to one of Claims 1 and 2, characterised in that the extraction is performed at a temperature and pressure outside the supercritical region.

4. Process according to one of Claims 1 to 3, characterised in that the extraction is performed by direct contact of the acid with carbon dioxide.

5. Process according to one of Claims 1 to 4, characterised in that the extraction is performed on a solution of mercapto acid in a polar solvent.

6. Process according to Claim 5, characterised in that the polar solvent is chosen from the group composed of water, linear or branched C₁-C₅ monohydric alcohols, C₂-C₆ diols and C₃-C₆ polyols.

7. Process according to one of Claims 5 and 6, characterised in that the solvent is used in an amount of between 1 and 99 % by weight relative to the mercapto acid.

8. Process according to one of Claims 5 to 7, characterised in that the solution of mercapto acid has a mercapto acid concentration of between 0.1 mol/litre and 14 mol/litre.

9. Process according to one of Claims 1 to 8, characterised in that the extraction is performed in the presence of an inert gaseous compound.

10. Process according to one of Claims 1 to 9, characterised in that a mercapto acid, where appropriate a solvent, carbon dioxide in gaseous, liquid or solid form and, where appropriate, an inert gas are introduced into a reactor, it is arranged for the pressure and temperature conditions desired for the extraction to be obtained, the constituents are left in contact for 5 to 30 minutes, the carbon dioxide is drawn off, several extraction cycles are performed where appropriate, reintroducing carbon dioxide, reestablishing the temperature and pressure conditions desired for the extraction, leaving the constituents in contact and evacuating the CO₂, and the deodorised mercapto acid or a solution of deodorised mercapto acid is collected.

11. Process according to one of Claims 1 to 9, characterised in that the mercapto acid and, where appropriate, a solvent are introduced into a reactor, the reactor is filled with carbon dioxide and it is arranged for the pressure and temperature conditions desired for the extraction to be obtained, a washing flow with gaseous or liquid carbon dioxide is established for a specified period, the CO₂ is evacuated and the deodorised mercapto acid or a solution of deodorised mercapto acid is collected.

12. Process according to Claim 11, characterised in that the flow of carbon dioxide is at between 0.5 and 3 kg/h.

13. Process according to Claim 12, characterised in that the flow of carbon dioxide is at between 1 and 30 kg/h per kg of mixture subjected to the extraction.

14. Process according to one of Claims 1 to 9, characterised in that the extraction is performed in continuous fashion with carbon dioxide in liquid or gaseous form on a separating column.

15. Process according to one of Claims 10 to 14, characterised in that the carbon dioxide is recycled after being relieved of the malodorous compounds.

## Patentansprüche

1. Verfahren zur Desodorierung einer Mercaptosäure durch Extraktion des größten Teils der übelriechenden Zersetzungsprodukte, die in der der Behandlung unterzogenen Mercaptosäure enthalten sind, dadurch gekennzeichnet, daß die behandelte Mercaptosäure die Formel
HS - A - COOH (I)
hat, in der A
- das zweiwertige Radikal
-(CH₂)ₙ-
worin n eine ganze Zahl zwischen 1 und 4 ist,
- das zweiwertige Radikal 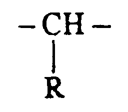 worin R ein lineares oder verzweigtes C₁-C₃-Alkylradikal ist, oder
- das zweiwertige Radikal 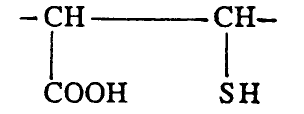 ist, und daß die Extraktion mit Hilfe von Kohlendioxid in gasförmigem, flüssigem oder festem Zustand vorgenommen wird.

2. Verfahren nach Anspruch 1, dadurch gekennzeichnet, daß die behandelte Mercaptosäure Thioglycolsäure oder Thiomilchsäure ist.

3. Verfahren nach einem der Ansprüche 1 oder 2, dadurch gekennzeichnet, daß die Extraktion bei einer Temperatur und einem Druck vorgenommen wird, die außerhalb des überkritischen Bereichs liegen.

4. Verfahren nach einem der Ansprüche 1 bis 3, dadurch gekennzeichnet, daß die Extraktion durch direkten Kontakt der Säure mit Kohlendioxid vorgenommen wird.

5. Verfahren nach einem der Ansprüche 1 bis 4, dadurch gekennzeichnet, daß die Extraktion an einer Mercaptosäurelösung in einem polaren Lösungsmittel vorgenommen wird.

6. Verfahren nach Anspruch 5, dadurch gekennzeichnet, daß das polare Lösungsmittel aus der Gruppe ausgewählt ist, die aus Wasser, den linearen oder verzweigten C₁-C₅-Monoalkoholen, den C₂-C₆-Diolen und den C₃-C₆-Polyolen besteht.

7. Verfahren nach einem der Ansprüche 5 oder 6, dadurch gekennzeichnet, daß das Lösungsmittel in einer Menge von 1 bis 99 Gew.-%, bezogen auf die Mercaptosäure, verwendet wird.

8. Verfahren nach einem der Ansprüche 5 bis 7, dadurch gekennzeichnet, daß die Mercaptosäurelösung eine Konzentration an Mercaptosäure von 0,1 Mol/l bis 14 Mol/l hat.

9. Verfahren nach einem der Ansprüche 1 bis 8, dadurch gekennzeichnet, daß die Extraktion in Anwesenheit einer neutralen gasförmigen Verbindung vorgenommen wird.

10. Verfahren nach einem der Ansprüche 1 bis 9, dadurch gekennzeichnet, daß eine Mercaptosäure, ggf. ein Lösungsmittel, Kohlendioxid in gasförmigem, flüssigem oder festem Zustand und ggf. ein neutrales Gas in einen Reaktor eingeführt werden, die für die Extraktion gewünschten Druck- und Temperaturbedingungen hergestellt werden, das Ganze während 5 bis 30 Minuten in Kontakt gelassen wird, das Kohlendioxid abgezogen wird, ggf. mehrere Extraktionszyklen durchgeführt werden, indem Kohlendioxid nachgefüllt wird, indem die für die Extraktion gewünschten Temperatur- und Druckbedingungen wiederhergestellt werden, indem das Ganze in Kontakt gelassen wird und indem das CO₂ abgeführt wird, und die desodorierte Mercaptosäure oder eine desodorierte Mercaptosäurelösung gewonnen wird.

11. Verfahren nach einem der Ansprüche 1 bis 9, dadurch gekennzeichnet, daß die Mercaptosäure und ggf. ein Lösungsmittel in einen Reaktor eingeführt werden, der Reaktor mit Kohlendioxid gefüllt wird und die für die Extraktion gewünschten Druck- und Temperaturbedingungen hergestellt werden, während einer bestimmten Zeit ein Waschdurchsatz von gasförmigem oder flüssigem Kohlendioxid hergestellt wird, das CO₂ abgeführt wird und die desodorierte Mercaptosäure oder eine desodorierte Mercaptosäurelösung gewonnen wird.

12. Verfahren nach Anspruch 11, dadurch gekennzeichnet, daß der Kohlendioxiddurchsatz 0,5 bis 3 kg/h beträgt.

13. Verfahren nach Anspruch 12, dadurch gekennzeichnet, daß der Kohlendioxiddurchsatz 1 bis 30 kg/h pro kg der der Extraktion unterworfenen Mischung beträgt.

14. Verfahren nach einem der Ansprüche 1 bis 9, dadurch gekennzeichnet, daß die Extraktion kontinuierlich mit Kohlendioxid in flüssigem oder gasförmigem Zustand auf einer Trennsäule vorgenommen wird.

15. Verfahren nach einem der Ansprüche 10 bis 14, dadurch gekennzeichnet, daß das Kohlendioxid rezykliert wird, nachdem es von den übelriechenden Verbindungen befreit wurde.
